# EUROPEAN PATENT SPECIFICATION

(11) **EP 2 813 245 B1**
(45) Date of publication and mention of the grant of the patent: **16.10.2019**
(21) Application number: 14157887.2
(22) Date of filing: 05.03.2014
(51) Int. Cl.: A61K 41/00, A61K 45/06, A61K 31/197, A61K 33/40, A61P 31/12, A61P 35/00, A61P 35/02, A61P 31/18, A61P 9/10, A61K 31/17

(54) **USE OF CARBAMIDE PEROXIDE INJECTION IN PREPARATION OF VIRUS INACTIVATION MEDICAMENTS IN COMBINATION WITH PHOTODYNAMIC THERAPY**
VERWENDUNG VON CARBAMIDPEROXIDINJEKTION BEI DER HERSTELLUNG VON VIRUSINAKTIVIERUNGSARZNEIMITTELN IN KOMBINATION MIT PHOTODYNAMISCHER THERAPIE
UTILISATION D'INJECTION DE PEROXYDE DE CARBAMIDE POUR PRÉPARER LES MÉDICAMENTS D'INACTIVATION DU VIRUS EN COMBINAISON AVEC LA PHOTOCHIMIOTHÉRAPIE

(30) Priority: 22.03.2013 CN 201310093820
(43) Date of publication of application: 17.12.2014
(73) Proprietor: Wuxi Zhaozhen Radiation Technology Co., Ltd., Wuxi Jiangsu 214000 (CN)
(72) Inventor: SUN, Qiyin, Wuxi Jiangsu Province (CN); SUN, Keke, Wuxi Jiangsu Province (CN)
(74) Representative: dompatent von Kreisler Selting Werner - Partnerschaft von Patent- und Rechtsanwälten mbB

(56) References cited:
- EP-A1- 2 774 610
- EP-A2- 2 722 057
- WO-A1-2011/006263
- OH-CHOON KWON ET AL: "Fluorescence Kinetics of Protoporphyrin-IX Induced from 5-ALA Compounds in Rabbit Postballoon Injury Model for ALA-Photoangioplasty", PHOTOCHEMISTRY AND PHOTOBIOLOGY, vol. 84, no. 5, 1 September 2008 (2008-09-01), pages 1209-1214, XP055151514, ISSN: 0031-8655, DOI: 10.1111/j.1751-1097.2008.00325.x
- R. N. MANIFOLD ET AL: "Increased cutaneous oxygen availability by topical application of hydrogen peroxide cream enhances the photodynamic reaction to topical 5-aminolevulinic acid-methyl ester", ARCHIVES OF DERMATOLOGICAL RESEARCH, vol. 303, no. 4, 3 February 2011 (2011-02-03), pages 285-292, XP055151498, ISSN: 0340-3696, DOI: 10.1007/s00403-011-1128-x
- AGUINALDO SILVA GARCEZ ET AL: "Antimicrobial mechanisms behind photodynamic effect in the presence of hydrogen peroxide", PHOTOCHEMICAL AND PHOTOBIOLOGICAL SCIENCES, ROYAL SOCIETY OF CHEMISTRY, CAMBRIDGE, GB, vol. 10, 1 January 2011 (2011-01-01), pages 483-490, XP009176340, ISSN: 1474-905X
- LIU H -L ET AL: "Progress and perspective of photosensitizers for photodynamic therapy", CHINESE JOURNAL OF NEW DRUGS 20120630 CHINESE JOURNAL OF NEW DRUGS CO. LTD. CHN, vol. 21, no. 12, 30 June 2012 (2012-06-30) , pages 1346-1353, ISSN: 1003-3734
- BURCH S ET AL: "Treatment of canine osseous tumors with photodynamic therapy: a pilot study.", CLINICAL ORTHOPAEDICS AND RELATED RESEARCH APR 2009, vol. 467, no. 4, April 2009 (2009-04), pages 1028-1034, ISSN: 1528-1132
- ALLISON RON ET AL: "Photodynamic therapy for chest wall progression from breast carcinoma is an underutilized treatment modality", CANCER, vol. 91, no. 1, 1 January 2001 (2001-01-01), pages 1-8, ISSN: 0008-543X

## Description

### Technical Field

The invention relates to the field of medicament, particularly to the use of carbamide peroxide injection in photodynamic therapy.

### Background Art

Cardiovascular and cerebrovascular diseases are one of the main causes leading to death throughout the world, and cause 15 million people death each year. In Western countries, atherosclerosis scleratheroma is a main cause of cardiovascular and cerebrovascular diseases.

Cardiovascular and cerebrovascular diseases are the main cause of morbidity and death. The cause of cardiovascular and cerebrovascular diseases is that plaque formed in artery over time reduces the blood flowing to the specific organs including brain and cardiac muscle. In certain cases, the reduction of blood flow causes symptoms of transient ischemic attack, scelalgia or angina. If arterial obstruction becomes more serious, it may lead to the injury of brain, leg or cardiac muscle, and the injury is fatal.

A method for treating cardiovascular and cerebrovascular diseases and avoiding further tissue injury is carried out by invasively eliminating plaque, which is generally achieved by invasive surgery. Another alternative method is achieved by balloon angioplasty, which includes inserting blood vessel with a catheter. During the process, artery stents may also be implanted. When angioplasty treatment can not be used due to the type of the plaque, the plaque may be bypassed through grafting a new blood vessel around the plaque region during the vascular surgery or cardiosurgery. Angioplasty or bypass surgery may be unavailable for some patients, for example, the patients cannot receive such treatment due to advanced age or poor health, or the plaque is not suitable for any one of the above treatments. In such case, the patients must try to control the disease by medical administration, such as medications. Since the surgery treatment for arterial plaque is invasive, and has risk of complication, it is not suitable for all patients. Therefore, a minimally invasive method is required for reducing or eliminating the formation of plaque in artery. The US Patents No. 5657760, 5590657 and 5524620 have disclosed the non-invasive methods for treating the redundant substances in tissues and blood vessels, especially cardiac vessels. However, these methods are not suitable for reducing the plaque, rather that using in blood vascular system.

Radiotherapy and chemotherapy have certain therapeutic effect on leukemia treatment. However, most of the therapies adopting traditional chemotherapy have unsatisfied therapeutic effect on most leukemia, except on M3 leukemia.

In comparison with common chemotherapy, photodynamic therapy (PDT) is a new therapy developing recently, and can damage malignant cells by bio-photosensitization. Its principle is that some photosensitizers having high fluorescent quantum yield are injected into body, and the photosensitizers have affinity to leukemia cells higher than that to normal cells, so to retain in leukemia cells. Once irradiated with strong light, the photosensitizers absorb photons and jump to an excited state, and then transfer the energy to the surrounding oxygen molecules to generate singlet oxygen. Singlet oxygen is a highly toxic agent for cells, and has acting path including directly damage, that is, the singlet oxygen destroys the PDT sensitive cellular substructures, such as cell membrane, mitochondrion or lysosome, to cause the damage of the cells.

In comparison with the traditional chemotherapy, PDT has the advantages of relatively selection specificity, low toxic and side effects, no invasion, and no accumulative toxicity. Recent years, departments of health in many countries like America, Japan, Holland, and Canada have successively confirmed the legality of the new tumor therapy. At the same time, the PDT therapy received more attention in non-tumor therapy, and was used for removing harmful cells or tissues. PDT utilizes photochemical reaction and a series of physiological immune reaction triggered by the photochemical reaction to achieve the aim for treating malignant tumors, and has many important advantages: (1) good selectivity: photodynamic therapy performs the therapy by photochemical reaction excited by illumination, and is effective only in the region of illumination, and thus has almost no damage to normal tissues and cells outside the region of illumination; (2) low toxic and side effects: the photodynamic medicaments which enter into the tissues will trigger the phototoxic reaction to kill the tumor cells only when reaching a certain concentration and receiving sufficient light irradiation, no phototoxic reaction is triggered in the non-light irradiated part of the human body, thus the hematopoietic function, the immune function and the organ function in vivo are no affected, in other words, photodynamic therapy has no severe toxicity as radiotherapy and chemotherapy; (3) minimal invasion: laser can be introduced to pathological tissue by means of optical fibers, endoscopes, ultrasonographies and other interventional technologies for therapy, so as to avoid injuries and pains caused by thoracotomy and laparotomy; (4) synergy with other therapies; and (5) repeatable treatment: since photosensitizer itself has no toxicity, and tumor cells have no drug tolerance to photosensitizer, the photodynamic therapy may be used repeatedly.

The fundamental principle of photodynamic therapy is that: the photosensitizer selectively aggregates around the tumor tissues first, and is excited to the excited state under excitation of light with a specific wavelength; the photosensitizer in the excited state can excite the dissolved oxygen in the tissues to form singlet oxygen with high oxidizing property and reactive oxygen species (ROS) in other forms; and the reactive oxygen species has biotoxicity and can destroy biomacromolecules such as proteins and nucleic acids, so as to cause necrosis of the tumor cells. Currently tens of thousands of patients all over the world have received the treatment of the therapy. The photodynamic therapy can treat dozens of cancers, including esophageal carcinoma, lung cancer, cerebroma, head and neck cancer, ocular tumor, pharyngeal cancer, tumor of chest wall, breast carcinoma, mesothelioma of pleura, abdominal sarcoma, bladder carcinoma, gynecological tumor, rectal cancer, cutaneous cancer and the like.

In the process of developing the photodynamic therapy, the selection and the use of photosensitizer is the core element. Photosensitizer for the photodynamic therapy should meet the following conditions: (1) maximum absorption wavelength between 600-800 nm, and absorption between 400-600 nm as small as possible; (2) high singlet oxygen yield; (3) strong phototoxicity and weak dark toxicity; (4) higher retention ratio in malignant tumor tissues; (5) single component; (6) water-solubility; and (7) fluorescence.

Photodynamic therapy utilizes the photochemical reaction of the photosensitizer (PS) excited by laser, and the reaction product such as singlet oxygen, free radical and the like can kill cells. Different from three main traditional tumor treatment means including surgery, radiotherapy and chemotherapy, the photodynamic therapy has the advantages of selectivity, irreversible destruction of diseased tissues while causing no damage on peripheral normal cells. Application of the photodynamic therapy (PDT) in treatment of malignant tumors, especially superficial tumors (such as esophageal cancer, bladder carcinoma, oral squamous cell carcinoma, and malignant melanoma) is increasingly becoming the hot spot of clinical research in recent years. However, the photosensitizers used in current PDT therapy have many disadvantages, such as long retention time in skin, slow excretion, easily causing side effect, long light exposure avoiding time, poor selectivity and the like.

A photooxidation dynamic therapy is applied for treating tumors, that is, photosensitizer is irradiated with laser to generate oxygen free radical in tumor focus region, and further to kill tumor cells. The method has the advantages of high safety, less side effects, repeatable treatments, good compliance, wide anti-tumor spectrum, suitability for systemic treatments, and excellent therapeutic effect for many malignant tumors, especially for malignant tumors with superficial foci.

However, in application of the photooxidation dynamic therapy for treating tumor, the first generation of photosensitizer is generally haematoporphyrins, which have insensitivity to photoresponsenot, low targeting property and high phototoxicity, and requires dark treatment after administration causing great inconvenience for life, and interventional administration causing injury to the patients. The second generation of photosensitizer is generally porphin or its derivatives, which has the main features of long response wavelength, low phototoxicity, no requirement for avoiding light exposure after administration, administration by intravenous injection, and small injury to the patients, and disadvantages of insensitivity to photoresponse leading to unsatisfied killing effect on tumor cells, easy photodegradation, difficult storage and requirement for preparation before use.

For example, a mucosal drug delivery system for photodynamic diagnosis and therapy is disclosed in Chinese patent with publication No. CN1435261A, consists of gelling material of thermosetting agent, penetration enhancer and cellulose compounds, which is liquid at low temperature, and transforms to gel state at body temperature after being administered. Gelling agent refers to thick liquid or semi-solid preparation in the forms of solution, suspension, or emulsion prepared from medicaments and adjuvants which could form gel, and is suitable for topical application at skin or body cavity, such as nasal cavity, vagina and rectum. Gelling agent matrixes are divided into aqueous and oily matrixes, and the aqueous gelling agent matrixes generally consists of water, glycerol, or propylene glycol and cellulose derivatives, carbomer and alginates, gum tragacanth, gelatin, starch and the like.

A hydrophilic in situ gel preparation precursor capable of rapid dissolution is disclosed in US patent US20080069857 and includes two different polysaccharide materials, such as derivatives of HA, and cellulose compounds. However, the two different polysaccharide materials are preserved separately, and mixed in application for rapid cross-linking in water so as to form gel.

Photosensitizer kills tumor cells through above mentioned mechanisms, but has few injury to normal tissues. Comparing with the three traditional tumor treatment means including surgery, chemotherapy and radiotherapy, photodynamic therapy has good application prospect in treatment of tumor as photodynamic therapy has dual selectivity, i.e. directional irradiation effect of optical fibers and the retention effect of the medicament in tumor tissues.

With the development of the research for photodynamic therapy, the photodynamic therapy is found to realize selective damage to target tissues by the reactive oxygen species (ROS) generated in photodynamic reaction triggered by the combination of photosensitizer medicament, irradiation, and oxygen molecule in tissues. During the process of PDT, the photosensitizer absorbs light energy, and then transforms from a ground state to a triplet excited state having relatively long duration after experiencing a transient single excited state. The photosensitizer in the excited state can cause two types of photodynamic reactions. In one photodynamic reaction, the photosensitizer in the triplet excited state can directly react with cell membranes or some biomacromolecules, transfer a hydrogen atom (electron) to form free radical, which interacts with the tissue oxygen to generate ROS (type I reaction) capable of killing target cells. In the other photodynamic reaction, the photosensitizer in the triplet excited state also can directly transfer the energy to the oxygen molecule, to form an effective ROS, i.e. singlet oxygen, to kill the target cells (type II reaction). Type I reaction and type II reaction occur simultaneously, the ratio of them depends on the type of the photosensitizer, the substrate and the concentration of tissue oxygen, as well as the binding tightness of the photosensitizer with the substrate. In the whole process, the photosensitizer transforms to the excited state from the ground state, then returns back to the ground state, and acts as a catalyst. Sufficient concentration of the tissue oxygen is required for PDT, and the photosensitization can not occur in oxygen-deficient tissue regions. The density of photodynamic effect is influenced by oxygen content in tissues; for example, the photodynamic effect is reduced in the oxygen-deficient regions of the tumor tissues. In the photodynamic process, after the photosensitizer is irradiated, the oxygen pressure in the tissues reduces rapidly and significantly. The reduction of the tissue oxygen limits the photosensitivity reaction, thereby influencing the killing effect of PDT. As it can be seen, the content of the oxygen in the tissues, as one of the three main photodynamic factors, cannot be ignored in the practical application. In order to satisfy the requirement of the photodynamic action for oxygen, PDT adopting stepwise irradiations is performed by some scholars, and reoxidation of the tissues is allowing, so as to overcome the problem. Some other scholars use HBO to increase the oxygenation content in the tissues, as the adjuvant therapy of the photodynamic therapy, so as to increase the lethal effect on tumor cells directly and indirectly. Chinese Patent "Laser Irradiation Device With Hyperbaric Oxygen Chamber" (Patent No. CN85103131) discloses a device performing photodynamic therapy under hyperbaric oxygen environment for increasing oxygen content in tissues, to improve the effect of the photodynamic therapy. Chinese Patent "Assembly of Photodynamic Therapy" (Application No. 03104558.8 and Publication No. CN1438048A) discloses a device for increasing the concentration of tissue oxygen in the target site by using a transdermal negative pressure drug delivery therapy apparatus to improve the effect of the photodynamic therapy. Safe and efficient novel photosensitizers and new methods for increasing the oxygen content in target tissues have been sought in order to further increase oxygen content in target tissues and improve therapeutic effect of photodynamic therapy.

Traditional photodynamic therapy (PDT) utilizes the affinity of the photosensitizer to tumor, leukemia cells, blood vessel plaque, dermatosis and viruses, which is several times higher than that to the normal tissues, and then irradiates focus, cells or viruses with visible light having specific wavelength capable of being absorbed by the photosensitizer; after absorbing the light energy, the photosensitizer in the focus transforms O₂ to ¹O₂, which then causes apoptosis and/or necrosis of the pathological cells, so as to achieve therapeutic effect. However, due to poor tissue penetrability, difficult quantitative treatment and frequent lack of O₂ in tumors, the actual effect of clinical PDT is not very satisfactory; at the same time, in many cases, clinically, invasive and non-invasive intervention means are required in clinical to introduce light source into the pathological sites, which is hard for doctors and patients to accept.

OH-CHOON KWON ET AL: "Fluorescence Kinetics of Protoporphyrin-IX Induced from 5-ALA Compounds in Rabbit Postballoon Injury Model for ALA-Photoangioplasty",PHOTOCHEMISTRY AND PHOTOBIOLOGY, vol. 84, no. 5, September 2008, pages 1209-1214, discloses the beneficial effect of photodynamic therapy with 5-ALA (5-aminolevulinic acid) for the treatment of atheromatous plaque.

### Summary of the Invention

The invention is defined by the claims. Any subject-matter falling outside the scope of the claims is provided for information purposes only. Furthermore, any reference in the description to methods of treatment refers to the compounds, pharmaceutical compositions and medicaments of the present invention for use in a method of treatment of the human or animal body by therapy.

It is an object of the invention to provide a method capable of inactivating virus for efficiently treating diseases like blood vessel plaque, leukemia, AIDS, tumor and hepatitis without side effects.

The present invention provides the following technical solutions to solve above mentioned problems.

Carbamide peroxide injection for use in treating blood vessel plaque, leukemia, tumor, AIDS and hepatitis in humans, wherein said diseases are treated by generating sufficient amount of singlet oxygen, i.e., ¹O₂, using carbamide peroxide as substrate and a photosensitizer as catalyst, which is activated by the effect of a method using laser, and X-ray, wherein

Carbamide Peroxide+ photosensitizer → singlet oxygen + water,

wherein said carbamide peroxide is intravenously dripped at a dose of 18-80 mg/kg body weight.

Wherein, the application includes the following steps:
step 1, determining treating target regions of focus by performing systemic CT plain scan on ill body or imaging method, or suffusing said disease with systemic irradiation;
step 2, determining treatment plan by plotting treating target regions of the focus on a treatment planning system or systemically irradiating the target regions, and irradiating once;
step 3, allowing oral administration of 5-ALA at 20-80 mg/kg 2-6 h before irradiation, intravenously dripping carbamide peroxide at 80 mg/kg immediately before and after irradiation, irradiating focus once with 4-6 Gy immediately after dripping carbamide peroxide, and systemically irradiating once at 0.5-1.0 Gy;
step 4, performing the treatment course 4-6 times according to the different types of the diseases; and
step 5, reexamining with CT or other imaging methods in 5-7 days after one treatment course and recording regression status.

Treatment principle: treatment mechanism is that the affinity of photosensitizer to big foam cells in blood vessel plaque, leukemia cells and virus is several times higher than that to normal tissues, the photosensitizer promotes the generation of more singlet oxygen by using **Carbamide Peroxide injection series** under the excitation of laser, X-ray, ultrasound, or medicament to destroy big foam cells, leukemia cells and virus, so as to achieve the purpose for treating above diseases.

### Beneficial Effects

1. Good therapeutic effect: especially in destroying mitochondrion in abnormal proliferative cells, and irreversibly killing malignant cells; and
2. Low side effects: photosensitizer concentrates in the focus and is activated in the focus target region, to allow the reaction to be centralized at the focus region, leukemia cells and virus surface, and less reactions on normal cells and sites.

The invention utilizes the affinity of photosensitizer to the blood vessel plaque focus, leukemia cells and the virus, which is several times higher than that to the normal tissues, to promote the generation of more singlet oxygen by using **Carbamide Peroxide injection series** under the excitation of X-ray or laser, to destroy the big foam cells, leukemia cells and virus, so as to achieve the purpose for treating above diseases.

The focus cells or virus are destroyed without serious side effects to the normal human tissues and organs.

### Description of Drawings

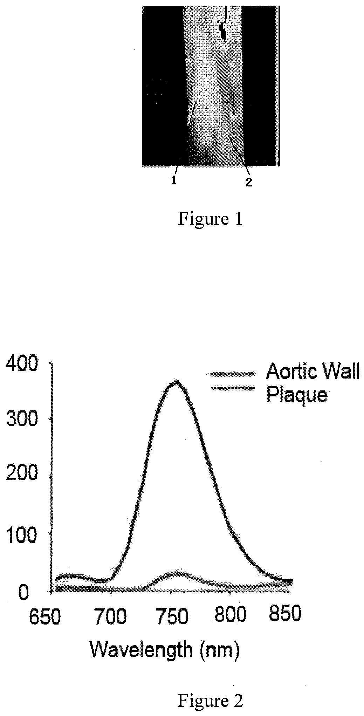

Figure 1: photosensitizer enters into human body and accumulates at blood vessel plaque; meanwhile, carbamide peroxide injection series are injected;
Figure 2: wavelength of X-ray used; and
Figure 3: the presence of plaque in the blood vessel of a rabbit was confirmed by CT examination, then the rabbit was given with the photosensitizer for oral administration and irradiated with high energy X-ray, CT examination was performed 5 days later, and it was found that the plaque is eliminated, and the therapeutic purposes were effectively achieved.

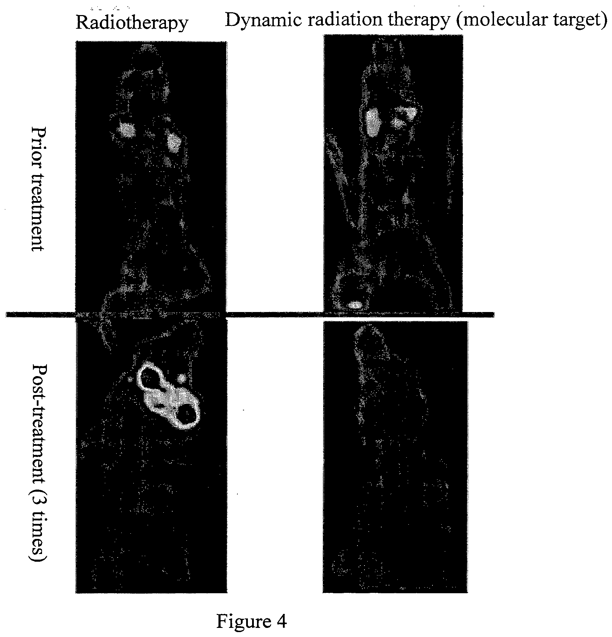

Figure 4: comparison of the effects before and after application of carbamide peroxide injection to the experimental rabbits;

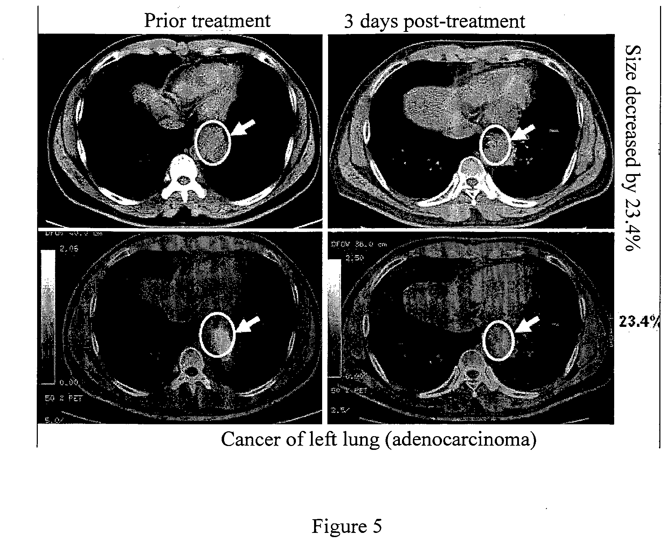

Figure 5: comparison of the effects of carbamide peroxide injection to lung cancer.

In the figures, 1 represents the plaque; and 2 represents the photosensitizer.

### Detailed Description of the Preferred Embodiments

Carbamide peroxide injection for treating disease, which is characterized in that, by virtue of the affinity of photosensitizers to blood vessel plaque focus, leukemia cells, tumor, AIDS and hepatitis virus-induced nervous system disease, which is several times higher than that to normal tissues, focus cells or virus are destroyed without side effects to normal human tissues and organs by generating sufficient amount of singlet oxygen, i.e., ¹O₂, with the endogenous and/or exogenous carbamide peroxide injection as substrate and with the photosensitizer as catalyst, which is activated by the effect of a method using laser or X-ray, wherein

Carbamide Peroxide+ photosensitizer → singlet oxygen + water,

wherein said carbamide peroxide is intravenously dripped at a dose of 18-80 mg/kg body weight.

Wherein, the application includes the following steps:
step 1, determining treating target regions of focus by performing systemic CT plain scan on ill body or imaging method, or suffusing said disease with systemic irradiation;
step 2, determining treatment plan by plotting treating target regions of the focus on a treatment planning system or systemically irradiating the target regions, and irradiating once;
step 3, allowing oral administration of 5-ALA at 20-80 mg/kg 2-6 h before irradiation, intravenously dripping carbamide peroxide at 80 mg/kg immediately before and after irradiation, irradiating focus once with 4-6 Gy immediately after dripping carbamide peroxide, and systemically irradiating once at 0.5-1.0 Gy;
step 4, performing the treatment course 4-6 times according to the different types of the diseases; and
step 5, reexamining with CT or other imaging methods in 5-7 days after one treatment course and recording regression status.

### Embodiment 1: Treatment of blood vessel plaque

### 1. Experimental method:

(1) Male New-Zealand Experimental rabbits each having body weight of 3-4 kg were fed with feed containing 1% cholesterol and lard for 11 weeks till the appearance of atherosclerotic plaque in bilateral fundus vessels and aorta.
(2) Experimental rabbits were anesthetized with ketamine and thiopental sodium.
(3) Treating target region of aorta plaque was determined with systemic CT plain scan and CTA enhancement.
(4) Treatment plan was determined by plotting treating target region of aorta plaque on a treatment planning system, irradiation was performed once, and normal feeding was recovered.
(5) 5-ALA (80 mg/kg in saline 5 mL) was intraperitoneally injected 2 h before irradiation, carbamide peroxide (injection containing H₂O₂, 80 mg/kg) in saline 5 mL was injected slowly at auricular vein immediately before irradiation, and the treatment plan was performed immediately after the injection of carbamide peroxide.
(6) Reexamination with CTA was performed 5-7 days after irradiation, and the regression of blood vessel plaque was recorded.

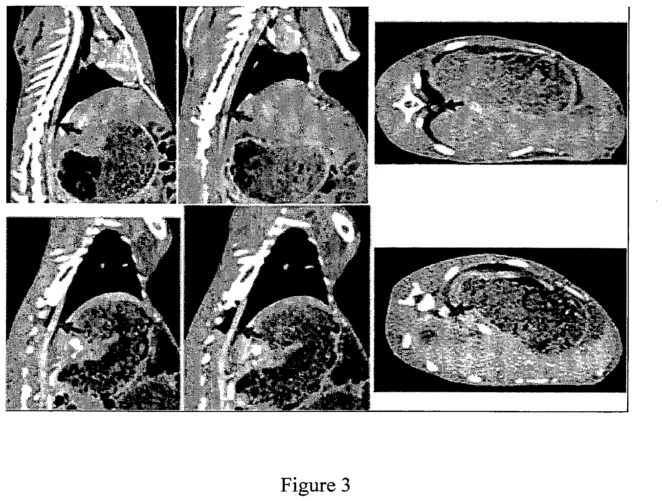

The New-Zealand rabbit model of blood vessel plaque is a common animal experimental model. New-Zealand rabbit was fed with high fat and high cholesterol for three months, and the formation of thoracic aorta blood vessel plaque was shown by examination with CTA angiography and MR. The blood vessel plaque was taken as the target region and positioned, and a radiotherapy plan was made. 1.5 h after intraperitoneal injection of 5-ALA (80 mg/kg body weight), the substrate was intravenously dripped, and the irradiation to the blood vessel plaque was performed once with 5 Gy. The New-Zealand rabbit was continually fed for 1 week. On the seventh day after the treatment, examination with CTA angiography and MR detection was performed again, and the result shows that the thoracic aorta blood vessel plaque completely disappeared (Shown in Fig. 3).

**The use of carbamide peroxide injection series** can promote generation of more singlet oxygen to destroy big foam cells, leukemia cells and virus. The use for treating leukemia and viral diseases is also in the protection scope of the present application.

## Claims

1. A carbamide peroxide injection for use in treating blood vessel plaque, leukemia, tumor, AIDS and hepatitis in humans, wherein said diseases are treated by generating sufficient amount of singlet oxygen, i.e., ¹O₂, using carbamide peroxide as substrate and a photosensitizer as catalyst, which is activated by the effect of a method using laser or X-ray, wherein 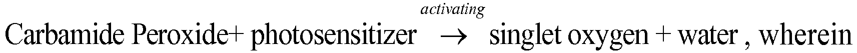 said carbamide peroxide is intravenously dripped at a dose of 18-80 mg/kg body weight.

2. The carbamide peroxide for use according to claim 1, **characterized in that**, said photosensitizer is 5-ALA.

3. The carbamide peroxide for use according to claim 2, **characterized in that**, said use includes the following steps:
step 1, determining treating target regions of focus by performing systemic CT plain scan on ill body or imaging method, or suffusing said disease with systemic irradiation;
step 2, determining treatment plan by plotting treating target regions of the focus on a treatment planning system or systemically irradiating the target regions, and irradiating once;
step 3, allowing oral administration of 5-ALA at 20-80 mg/kg 2-6 h before irradiation, intravenously dripping carbamide peroxide immediately before and after irradiation, irradiating focus once with 4-6 Gy immediately after dripping carbamide peroxide, and systemically irradiating once at 0.5-1.0 Gy;
step 4, performing the treatment course 4-6 times according to the different types of the diseases; and
step 5, reexamining with CT or other imaging methods in 5-7 days after one treatment course and recording regression status.

## Patentansprüche

1. Carbamidperoxid-Injektion zur Verwendung bei der Behandlung von Blutgefäßplaques, Leukämie, eines Tumors, von AIDS und Hepatitis bei Menschen, wobei die Krankheiten behandelt werden, indem eine ausreichende Menge Singulett-Sauerstoff, d. h. ¹O₂, erzeugt wird, wobei Carbamidperoxid als Substrat und ein Photosensibilisator als Katalysator verwendet werden, der durch die Wirkung eines Verfahrens aktiviert wird, bei dem ein Laser oder Röntgenstrahlung verwendet wird, wobei 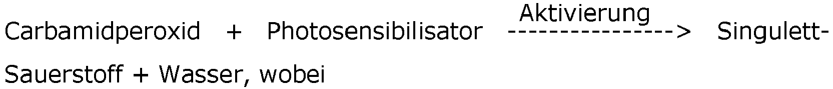 das Carbamidperoxid mit einer Dosis von 18-80 mg/kg Körpergewicht intravenös infundiert wird.

2. Carbamidperoxid zur Verwendung nach Anspruch 1, **dadurch gekennzeichnet, dass** es sich beim Photosensibilisator um 5-ALA handelt.

3. Carbamidperoxid zur Verwendung nach Anspruch 2, **dadurch gekennzeichnet, dass** die Verwendung die folgenden Schritte einschließt:
Schritt 1, das Bestimmen von Behandlungszielregionen des Herdes durch das Durchführen einer systemischen CT-Leeraufnahme des kranken Körpers oder eines Bildgebungsverfahrens oder das Durchdringen der Krankheit mit systemischer Bestrahlung;
Schritt 2, das Bestimmen eines Behandlungsplans durch das Aufnehmen von Behandlungszielregionen des Herdes in ein Behandlungsplanungssystem oder das systemische Bestrahlen der Zielregionen und ein einmaliges Bestrahlen;
Schritt 3, das Zulassen einer oralen Verabreichung von 5-ALA mit 20-80 mg/kg 2-6 Stunden vor der Bestrahlung, das intravenöse Infundieren von Carbamidperoxid unmittelbar vor und nach der Bestrahlung, das einmalige Bestrahlen des Herdes mit 4-6 Gy unmittelbar nach dem Infundieren von Carbamidperoxid und das einmalige systemische Bestrahlen mit 0,5-1,0 Gy;
Schritt 4, das 4-6-malige Durchführen der Behandlungsphase gemäß den verschiedenen Krankheitstypen und
Schritt 5, ein erneutes Untersuchen mittels CT oder anderen Bildgebungsverfahren 5-7 Tage nach einer Behandlungsphase und ein Registrieren des Regressionsstatus.

## Revendications

1. Injection de peroxyde d'urée pour son utilisation dans le traitement d'une plaque de vaisseaux sanguins, d'une leucémie, d'une tumeur, du SIDA et de l'hépatite chez des humains, dans laquelle lesdites maladies sont traitées en générant une quantité suffisante d'oxygène singulet, c'est-à-dire, ¹O₂, à l'aide de peroxyde d'urée en tant que substrat et d'un photosensibilisateur en tant que catalyseur, qui est activé par l'effet d'un procédé utilisant un laser ou des rayons X, dans laquelle 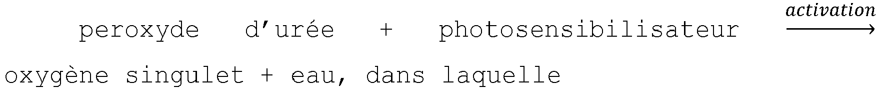 ledit peroxyde d'urée est injecté en goutte-à-goutte par voie intraveineuse à une dose de 18 à 80 mg/kg de poids corporel.

2. Peroxyde d'urée pour son utilisation selon la revendication 1, **caractérisé en ce que**, ledit photosensibilisateur est le 5-ALA.

3. Peroxyde d'urée pour son utilisation selon la revendication 2, **caractérisé en ce que**, ladite utilisation comporte les étapes suivantes :
étape 1, la détermination de régions cibles de traitement d'un foyer en réalisant un simple tomodensitogramme systémique sur un corps malade ou un procédé d'imagerie, ou par suffusion de ladite maladie avec une irradiation systémique ;
étape 2, la détermination d'un plan de traitement en traçant des régions cibles de traitement du foyer sur un système de planification de traitement ou en irradiant de façon systémique les régions cibles, et en irradiant une fois ;
étape 3, l'autorisation d'une administration orale de 5-ALA à 20 à 80 mg/kg, 2 à 6 h avant l'irradiation, l'injection intraveineuse en goutte-à-goutte de peroxyde d'urée immédiatement avant et après l'irradiation, l'irradiation du foyer une fois avec 4 à 6 Gy immédiatement après l'injection en goutte-à-goutte de peroxyde d'urée, et l'irradiation systémique une fois à 0,5 à 1,0 Gy ;
étape 4, la réalisation du traitement 4 à 6 fois selon les différents types des maladies ; et
étape 5, le réexamen par tomodensitogramme ou d'autres procédés d'imagerie dans les 5 à 7 jours après un traitement et l'enregistrement du statut de régression.
